# NEW EUROPEAN PATENT SPECIFICATION

(11) **EP 0 893 977 B2**
(45) Date of publication and mention of the opposition decision: **28.10.2009**
(45) Mention of the grant of the patent: 19.04.2006
(21) Application number: 97950595.5
(22) Date of filing: 07.11.1997
(51) Int. Cl.: A61F 2/06

(54) **Variable flexibility stent**
Stent mit variabler Flexibilität
Extenseur à flexibilité variable

(30) Priority: 07.11.1996 US 29936 P
(43) Date of publication of application: 03.02.1999
(73) Proprietor: Medtronic Instent Inc., Eden Prairie, MN 55346 (US)
(72) Inventor: GLOBERMAN, Oren, 58543 Holon (IL); BEYAR, Mordechay, 38900 Caeseria (IL); BEYAR, Rafael, 34751 Haifa (IL)
(74) Representative: Hughes, Andrea Michelle
(86) International application number: PCT/US1997/020695
(87) International publication number: WO 1998/022159

(56) References cited:
- EP-A- 0 800 801
- WO-A-93/15661
- WO-A-97/25937
- WO-A-97/32543
- WO-A-98/18404
- US-A- 5 443 496
- US-A- 5 449 373
- US-A- 5 749 919

## Description

### FIELD OF THE INVENTION

This invention concerns a novel stent. More particularly, this invention concerns a stent having variable flexibility and stiffness.

### BACKGROUND OF THE INVENTION

A common characteristic of many stents in the market is longitudinal axis symmetry. Some of the stents, such as the Medtronic WIKTORT™ stents and the Medtronic InStent CARDIOCOILT™ stents, have a spiral structure. Such spiral structures have a cyclic helical coil element called "pitch." Other stents, such as the ACS MULTILINK™ stent, the Johnson & Johnson PALMAZ-SCHATZ™ stent, and the Medtronic Instent beSTENT™, are designed with a cyclic mesh element. Further stents such as the Johnson & Johnson Articulated Stent and the NIR stent have a double cyclic structure, that is, odd and even longitudinal elements where an odd element comes after an even element, and all the odd elements are identical and all the even elements are identical.

All the above stents have in common the characteristic of the uniformity of flexibility or durability for bending along the length of the stent.

To facilitate bending but at the same time maintain form stability, US 5 499 373 A describes a stent of almost uniform rigidity along its length but with two less rigid transitions areas of increased flexibility on either side of the middle section of the stent.

To prevent breakage in the central area of the stent, WO 95/18585, for example, has proposed a stent which is more rigid in that area than in the other parts of the stent.

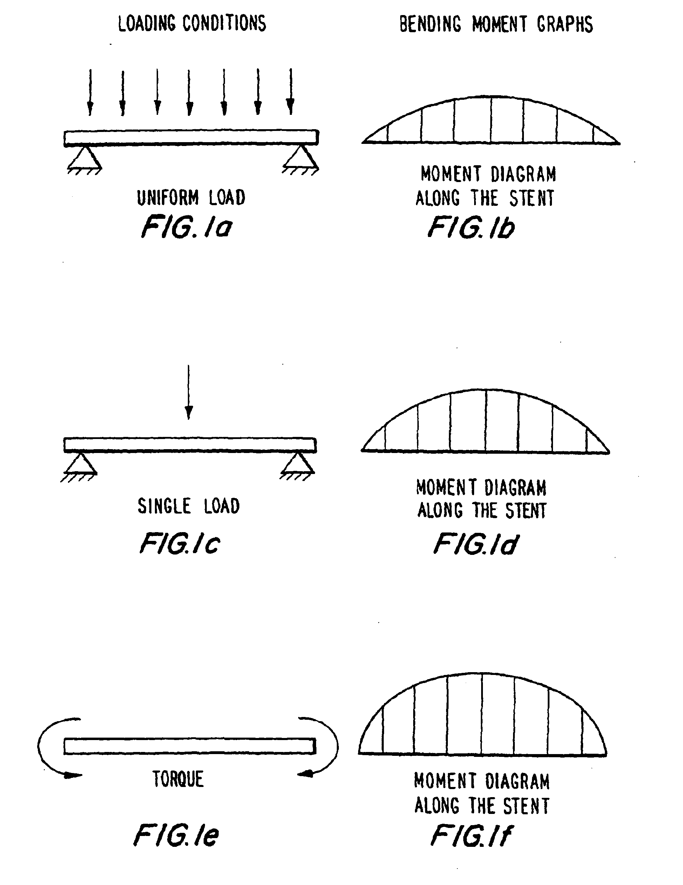

Tests done on long stents loaded with bending stresses reveal a large bending moment in the central area of the stent. (See, for example, Figs. 1a-1f.) Such stresses may cause fatigue and even fracture at the center of the stent. On the one hand, significant stent flexibility is required during deployment while, on the other hand, significant rigidity is required in the expanded state of the stent.

Since the bending moment increases from zero at the ends of the stent to a maximum value at the center, it has been determined that there is a need for a stent having a significant durability for bending at the exact place where the durability is required, that is, at the middle of the stent.

### OBJECTS OF THE INVENTION

It is an object of this invention to provide a novel stent which does not have the disadvantages inherent in known stents.

It is also an object of this invention to provide a stent having variable flexibility and/or durability and/or stiffness.

It is further object of the invention to provide a stent having a large or significant durability for bending, i.e., rigidity or stiffness, at the middle portion of the stent.

According to the present invention, there is provided a stent as defined in claim 1. Preferred embodiments are further specified in the dependent claims.

According to the invention the rigidity gradually increases from low rigidity at the ends to maximum rigidity at the center It provides simultaneously maximum resistance to bending failure and the maximum of flexibility at the ends for ease of insertion in contrast to the previously known stents.

These and other objects of the invention will become apparent to one skilled in the art from the discussion below.

### SUMMARY OF THE INVENTION

The present invention provides for a novel stent which overcomes many of the disadvantages associated with various prior art stent designs which rely upon a stent construction which provides for a uniformly rigid construction or a uniform alternating rigid/less rigid construction. The invention provided for herein is directed to a stent having variable flexibility and moment along its length. The stent comprises portions of the stent having different bending and durability characteristics, as designed. In one embodiment of the invention, the members of the stent have components that have been designed to impart different load characteristics.

### BRIEF DESCRIPTION OF THE DRAWINGS

Figs. 1a-1f are diagrams of loading studies of various stents;
Fig. 2 is an oblique view of one embodiment of the invention; and
Fig. 3 is a plan view of another embodiment of the invention.

### DESCRIPTION OF THE PREFERRED EMBODIMENT

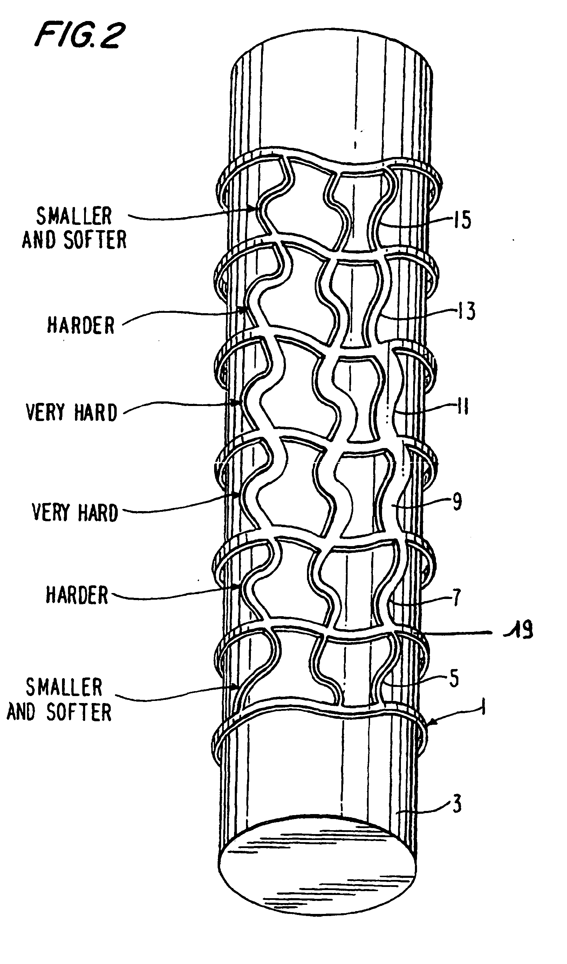

The invention can perhaps be better appreciated by making reference to the drawings. In Fig. 2, a stent 1 is positioned on the outer surface of a catheter 3. Stent 1 in this embodiment comprises a mesh lattice construction having sections 5, 7, 9, 11, 13, and 15 which are of varying degrees of flexibility or stiffness. For example, end sections 5 and 15 are comprised of smaller diameter cross members and therefore, a less rigid mesh, inner sections 7 and 13, are comprised of larger diameter cross members and a more rigid mesh, and center sections 9 and 11 are comprised of even larger diameter cross members and still a more rigid mesh.

Annular cross members 19 are shown in Fig. 2 to be substantially similar in size, both in length and cross-section. However, it is within the scope of the invention that cross members 19 can vary in size, especially cross-section, and impart different rigidity to stent 1. Similar to the discussion above for sections 5, 7, 9, 11, 13, and 15, cross members 19 could have a larger cross-section near the middle of stent 1 and increasingly smaller cross-sections toward the ends of stent 1.

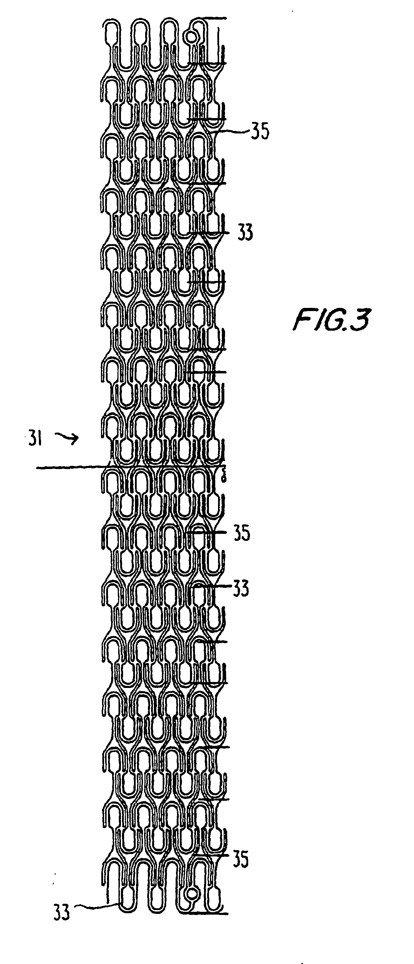

Another embodiment of the invention is represented in Fig. 3 by the partial latticework section 31 of a balloon expandable stent where annular members 33 expand as a dilatation balloon is expanded within the stent. Cross members 35 will continue to "link" members as the stent expands. While annular member 33 can be of similar size and rigidity, according to the invention cross members 35 will differ in size, e.g., thickness or even length, over the length of stent 31, to provide desired characteristics. It is anticipated that, as described before for the embodiment of Fig. 2, cross members 35 will be more rigid toward the longitudinal center of stent 31 and less rigid toward the ends.

The actual dimensions of stents according to the invention will vary dependent upon intended use and the characteristics to be imparted. For example, for a stent intended for cardiovascular use and having an expanded length of from about 3 to 10 cm, the annular members and/or cross members may each have a cross-sectional profile of from about 0.01 to 0.10 cm, varying as discussed above.

The stents according to the invention can be comprised of a variety of materials known to be useful in fabricating stents, such as stainless steel, nitinol, tantalum, and/or other metals or alloys. If the stent is a mesh, the mesh can be formed, stamped, or etched to vary the size and/or diameter of the mesh components. Alternatively, the mesh could be formed from component members of different diameters that are soldered or welded together or component members of the stent may be of different composition to vary the stiffness of the stent in the longitudinal direction Fabrication is discussed more below.

It is within the scope of the invention that a stent having variable stiffness could be comprised of other than a mesh.

A primary characteristic of stents according to the invention is that the moment diagrams, such as are shown in Figs. 1a-1f, will be modified to avoid an unacceptable load, or peak, in the middle. Thus, in the stents according to the present invention the load will be distributed more evenly across the entire length of the stent.

The manufacture of stents according to the presentation invention may be accomplished via any of a variety of methods.

In one method of fabrication of a patterned etched cylinder a wire mesh is formed from a flat planar surface and then its two opposite edges are fused to create a cylinder. This method, however, suffers a basic disadvantage in that the presence of the fusing line creates a weakened area along the longitudinal axis of the stent, which is potentially subject to fatigue and breakage. Therefore, it is preferable for the stent to be formed from a more uniform piece of material to avoid this potential problem.

There are also two alternative methods for imaging the desired pattern, i.e, the location of points, undulating connectors, ring and connecting segments, etc., as may be required by a particular stent design onto a continuous cylinder, without the need of fusing into a cylinder after forming of the design. These methods, a film contact imaging method and a laser scanning method, are disclosed in copending U.S. patent application Serial No. 08/543,337, filed October 16, 1995.

As has been described in more detail in said co-pending application, the film contact imaging method is carried out using an elliptical mirror which reflects ultraviolet light from an ultraviolet light source. The ultraviolet light source is located at one focal point of the elliptical mirror and illuminates through a slit of narrow aperture (which eliminates scattered light). The slit or aperture is located at the other focal point of the elliptical mirror to allow for high density power illumination from the ultraviolet source. Rays of ultraviolet light are thus reflected off of the elliptical mirror to pass through the slit or aperture and onto a moving film. The slit extends parallel to the longitudinal axis of a hollow tube or cylinder. The film carries the design sought to be provided onto the surface of the tube or cylinder.

The film is in contact with the hollow cylinder. The hollow cylinder is made of material which is to be fabricated into the stent of the present invention. The film serves as a mask or template, being transparent to ultraviolet light in some areas and opaque to ultraviolet light in others in the predefined stent pattern. The cylinder is coated with an appropriate material (a photoresist) for a photo-etching process. As ultraviolet light is transmitted onto the film through the slit, the film moves past the cylinder while the cylinder rotates. The rotation of the cylinder is correlated with the movement of the film to appropriately image the pattern on the film around and onto the cylinder. As a result, ultraviolet light passing through W-transparent portions of the film template will strike the cylinder in the desired pattern to photoetch the appropriate configuration onto the cylinder. An acid treatment is then used to remove the areas which were struck by the UV light. In general, the chemical aspects of the system are similar to that used in the manufacturer of computer chips, i.e., photoresist, masking, acid, etc.

It should be pointed out that variations on this design will be understood by those of ordinary skill in the art. For example, in the presence of a sufficiently high powered light source, usage of an elliptical mirror is not essential.

The second, and preferred, method, which is also described in more detail in said co-pending application, uses a laser scanning system. The system consists of a cylinder or tube to be etched, a laser, the laser optics (containing beam components and modulator), and a dynamic deflector (such as a rotating mirror, a polygon, or any other known scanning deflector). The system is based upon a well-known flat bed scanning system. The cylinder is coated with a photoresist, or material suitable for photoetching. A laser is selected of the appropriate power and wavelength suitable for stimulating the photoresist in use. For example, for an ablation method, the laser can be high powered IR laser diode; for a photoresist sensitive to visible light, the laser can be a laser in the visible range or for a conventional W photoresist, an Eximer laser or third (or higher) harmonic generation Nd:YAG/Nd:YLF laser can be used. The laser beam is shaped by an appropriate optical system, and modulated by direct modulation in the case of an IR laser diode, with AOM (an Acoustic Optical Modulator) in the case of a CW laser in the visible, or by a vibrating mirror in the case of a W laser.

The laser beam from the laser hits a deflector device which can be a rotating mirror, a polygon mirror, or other known scanning device. The beam emerges from the deflector, passing through a scan lens and is focused on the cylinder. The cylinder, which is coated with a photoresist, rotates about its longitudinal axis at a constant angular velocity, while the beam scans back and forth. The modulation of the laser beam allows writing a computer imaging file directly on the cylinder without the need of intermediate media (e.g. film). The laser scanning velocity is correlated to the cylinder angular velocity, and is determined by the energy required for exposure of the photoresist.

It is contemplated that the stents of the present invention may be constructed in any of a number of configurations and arrangements known in the art so long as the primary requirement of the invention is met, that being - that the stent be constructed in such a manner that the end product stent is provided with a varying degree of flexibility and stiffness along the length of the stent.

It will thus be seen that the objects set forth above, among those made apparent from the preceding description, are efficiently attained and, since certain changes may be made in the constructions set forth without departing from the scope of the invention as defined in the appended claims, it is intended that all matter contained in the above description and shown in the accompanying drawings shall be interpreted as illustrative and not in a limiting sense.

## Claims

1. A stent having a different bending flexibility along its length wherein the stent comprises component members has a center portion of high rigidity and end portions (5,15) of relatively low rigidity, wherein the component members comprise a tubular member consisting essentially of annular members (33) intended to expand and cross members (35) having ends, the ends of each cross member being attached to an annular member, **characterized in that** said stent has a gradually increasing rigidity from that end portions (5,15) towards the center portion wherein the stent, to vary gradually, in the longitudinal direction, the rigidity of the stent, is comprised of cross members having different dimensions or different compositions.

2. The stent of Claim 1, wherein the component members are forming a mesh.

3. The stent of Claim 1, wherein the component members are fabricated out of a material selected from the group comprising stainless steel, nitinol, titanium, tantalum, and other metals and metal alloys.

4. The stent of Claim 1, wherein the component members have been fabricated by a method selected from the group comprising forming, stamping and etching.

5. The stent of Claim 1, wherein the component members have been fabricated using a film contact imaging method.

6. The stent of Claim 1, wherein the component members have been fabricated using a laser cutting method.

## Patentansprüche

1. Stent mit einer unterschiedlichen Biegsamkeit entlang seiner Länge, wobei der Stent Einzelelemente umfasst und einen Mittenabschnitt hoher Steifigkeit und Endabschnitte (5, 15) relativ niedriger Steifigkeit besitzt, wobei die Einzelelemente ein rohrförmiges Element umfassen, das im Wesentlichen aus ringförmigen Elementen (33), die dazu gedacht sind, sich auszudehnen, und Querelementen (35) mit Enden besteht, wobei die Enden jedes Querelements mit einem ringförmigen Element verbunden sind, **dadurch gekennzeichnet, dass** der Stent eine von den Endabschnitten (5, 15) in Richtung des Mittenabschnitts allmählich zunehmende Steifigkeit hat, und wobei der Stent, um die Steifigkeit des Stents in der Längsrichtung allmählich zu verändern, aus Querelementen unterschiedlicher Abmessungen oder unterschiedlicher Zusammensetzungen besteht.

2. Stent nach Anspruch 1, wobei die Einzelelemente ein Netz bilden.

3. Stent nach Anspruch 1, wobei die Einzelelemente aus einem Material hergestellt sind, das aus der Gruppe ausgewählt ist, die rostfreien Stahl, Nitinol, Titan und weitere Metalle und Metalllegierungen umfasst.

4. Stent nach Anspruch 1, wobei die Einzelelemente durch ein Verfahren hergestellt wurden, das aus der Gruppe ausgewählt ist, die Formen, Stanzen und Ätzen umfasst.

5. Stent nach Anspruch 1, wobei die Einzelelemente unter Verwendung eines Filmkontaktabbildungsverfahrens hergestellt wurden.

6. Stent nach Anspruch 1, wobei die Einzelelemente unter Verwendung eines Laserschneidverfahrens hergestellt wurden.

## Revendications

1. Extenseur ayant une flexibilité de courbure différente le long de sa longueur en quoi l'extenseur comprend des membres composants ayant une portion centrale à haute rigidité et des portions d'extrémité (5, 15) à rigidité relativement faible, dans lequel les membres composants comportent un membre tubulaire consistant essentiellement de membres annulaires (33) conçus pour se détendre et de membres transversaux (35) ayant des extrémités, l'extrémité de chaque membre transversal étant rattachée à un membre annulaire, **caractérisé en ce que** ledit extenseur présente une rigidité augmentant graduellement desdites portions d'extrémité (5, 15) vers la portion centrale, l'extenseur, pour varier graduellement la rigidité de l'extenseur, dans la direction longitudinale, comportant des membres transversaux ayant des dimensions différentes ou des compositions différentes.

2. Extenseur de la revendication 1, dans lequel les membres composants forment une maille.

3. Extenseur de la revendication 1, dans lequel les membres composants sont fabriqués d'une matière sélectionnée parmi le groupe comprenant de l'acier inoxydable, du nitinol, du titane, du tantale, et d'autres métaux et alliages de métaux.

4. Extenseur de la revendication 1, dans lequel les membres composants ont été fabriqués par une méthode sélectionnée parmi le groupe comprenant le formage, l'estampage et le gravage.

5. Extenseur de la revendication 1, dans lequel les membres composants ont été fabriqués en utilisant une méthode d'imagerie par contact sur film.

6. Extenseur de la revendication 1, dans lequel les membres composants ont été fabriqués en utilisant une méthode de coupe au laser.
